# DEMANDE DE BREVET EUROPEEN

(11) **EP 4 807 360 A1**
(43) Date de publication de la demande: **16.09.2026**
(21) Numéro de dépôt: 26159903.9
(22) Date de dépôt: 20.02.2026
(51) Int. Cl.: G01N 33/24, C12Q 1/04, C12Q 1/64, G01N 17/00

(54) **DISPOSITIF D'ANALYSE DE BIOFILM DANS UN MILIEU POREUX**

(30) Priorité: 14.03.2025 FR 2502611
(71) Demandeur: IFP Energies nouvelles, 92500 Rueil-Malmaison (FR)
(72) Inventeur: GUILLON, Valentin, 92852 RUEIL-MALMAISON CEDEX (FR); VINDRET, Alexis, 92852 RUEIL-MALMAISON CEDEX (FR); POIRIER, Simon, 92852 RUEIL-MALMAISON CEDEX (FR)
(74) Mandataire: IFP Energies nouvelles

(57) **Abrégé**

L'invention concerne un dispositif d'analyse du comportement de biofilms au sein d'un milieu poreux , avec :
- une enceinte définissant une cavité pour le milieu poreux et qui comprend au moins une entrée d'injection de fluide et au moins une sortie d'évacuation de fluide,
- un moyen d'injection du fluide connecté fluidiquement à ladite entrée et comprenant un composant 15 logé dans l'enceinte et définissant une surface 16 d'une première porosité en regard du milieu poreux, qui est recouverte d'un filtre 17 comportant au moins trois couches de matériau superposées, dont une première couche 17a d'un matériau présentant une deuxième porosité inférieure à la porosité dudit composant et qui est disposée entre une deuxième et une troisième couches 17b,17c de matériaux identiques ou différents entre eux et qui présentent des troisièmes porosités supérieures à la deuxième porosité .

## Description

### Domaine technique

L'invention concerne le domaine de l'analyse des biofilms dans des milieux poreux. Plus particulièrement, elle concerne un dispositif d'analyse d'un biofilm ainsi que son procédé de mise en œuvre.

Dans les milieux réels (aquifères, réservoirs de gaz ou pétroliers), des bactéries peuvent survivre et croitre, entrainant la formation de biofilm ayant des effets très divers et colonisant ainsi le milieu poreux et les abords des puits de forage. Cette croissance de biofilm peut impacter des procédés très variés comme la géothermie, la dépollution des sols, la captation d'eau potable dans les aquifères, l'extraction pétrolière et gazière, le stockage géologique des gaz (CO₂, gaz naturel, H₂,...).

Parmi ces applications, on peut citer l'exploration et l'exploitation de puits d'hydrocarbures, particulièrement avec récupération assistée d'hydrocarbures (ou EOR selon la terminologie anglo-saxonne pour « Enhanced Oil Recovery »). On peut aussi citer la récupération géothermique de fluides souterrains dans des roches poreuses, ou encore le stockage de gaz du type CH₄, H₂, CO₂ ou du gaz naturel, dans des roches poreuses, pour leur utilisation ultérieure (H₂, CH₄, gaz naturel) ou pour leur piégeage (CO₂).

Dans ces trois cas de figures, on est amené à injecter ou réinjecter des fluides, gaz ou liquide, à travers des puits de forage, dans des roches poreuses, et, dès le départ de l'exploitation ou du fait même de ces injections/réinjections de fluides qu'implique leur exploitation, les roches poreuses et les puits d'injection/production, peuvent être le siège de proliférations de micro-organismes.

Or l'accumulation ou l'activité des micro-organismes dans ces roches réservoirs est préjudiciable à leur exploitation. En effet, il a été observé que ces micro-organismes tendent à former des biofilms par la production de polymères extracellulaires, causant une réduction significative du volume poral disponible des matériaux poreux colonisés par ces bactéries. (On comprend dans tout le présent texte par volume poral le volume du matériau poreux qui est accessible à un fluide). On parle alors de colmatage biologique, ce qui pose de multiples problèmes : réduction du volume disponible pour le stockage de fluide, pertes de charge pouvant devenir rédhibitoires dans le cas d'injection de fluide, une partie de la porosité de la roche devenant inaccessible aux fluides, réservoir de micro-organismes qui pourront par la suite recoloniser le milieu. Ces biofilms peuvent aussi avoir des effets positifs, il a par exemple été montré qu'ils pouvaient modifier ou améliorer l'étanchéité des « casings » (cimentations) de puits d'injection/production en obturant les microfissures qui peuvent s'y développer. Parmi les micro-organismes généralement impliqués se trouvent notamment des bactéries sulfato-réductrices, qui utilisent le soufre des ions SO₄, lesquels sont transformés en ions sulfites, en sulfure ou en H₂S, ce qui favorise en outre la corrosion des circuits, matériaux et équipements utilisés pour l'injection de fluide dans ces roches poreuses.

L'étude expérimentale du développement et du comportement en milieu poreux de ces microorganismes (bactéries notamment) et biofilms permet de mieux comprendre les différents mécanismes de croissance et de déplacement des microorganismes et du biofilm, et ainsi de pouvoir étudier leurs effets. A partir des résultats obtenus, on peut envisager les moyens de remédiation ou de contrôle de ces phénomènes appropriés.

### Technique antérieure

Cette problématique de formation de ces biofilms a été observée dans des applications pétrolières, et documentée par exemple dans la publication « Action of glutaraldehyde and nitrite against sulfate-reducing bacterial biofilms », de Gardner LR, Stewart PS (J.Ind. Microbiol. Biotechnol. (2002) 29 : 354 - 360).

Elle a été également documentée dans le cadre de la réinjection d'eau dans des roches poreuses visant une application géothermique, par exemple dans la publication « Biofilm Forming Bacteria during Thermal Water Reinjection », de Máté Osvald, Gergely Maróti, Bernadett Pap, and János Szanyi Geofluids (Indawi Geofluids, Volume 2017, Article ID 9231056, January 2017).

Les techniques expérimentales existantes aux laboratoires pour l'étude du comportement des biofilms en milieu poreux se font généralement à pression imposée pour reproduire le processus naturel. Toutefois, ces techniques se heurtent à la contamination et la croissance de biofilms dans les zones d'injection, réduisant ainsi l'injectivité et pouvant amener à un arrêt de l'écoulement dans le milieu poreux.

Une première solution a été apportée dans le brevet FR3121452, qui propose un procédé d'analyse du comportement d'un biofilm au sein d'un milieu poreux en deux étapes :
a) on injecte un fluide à une entrée d'une enceinte comprenant le milieu poreux à un débit constant pour générer le biofilm ou pour éliminer au moins partiellement le biofilm, le fluide pouvant ressortir de l'enceinte par une première sortie de l'enceinte, en amont du milieu poreux, et
b) on contrôle un gradient de pression entre l'entrée et une deuxième sortie de l'enceinte de manière à imposer un écoulement à gradient de pression prédéterminé dans le milieu poreux, la deuxième sortie étant située en aval du milieu poreux.

Ce contrôle de gradient de pression entre l'amont et l'aval du milieu poreux, dans le sens de circulation du fluide, s'est avéré pertinent pour améliorer la représentativité des résultats obtenus au laboratoire, en améliorant l'injectivité des fluides dans le dispositif d'analyse.

Cependant, il reste susceptible d'améliorations. En effet, l'injection de différents fluides (dont notamment des solutions contenant des micro-organismes, des solutions contenant des nutriments ou encore des solutions contenant un biocide selon différents protocoles d'analyse) dans la cellule du dispositif d'analyse contenant un échantillon représentatif du matériau poreux dont on veut analyser le comportement peut continuer à se heurter à deux risques potentiels :
- d'une part, un risque de contamination par des micro-organismes et/ou des biofilms non voulue/ non maîtrisée du matériau poreux à analyser
- d'autre part, un risque de « remontée » de micro-organismes et/ou de biofilm depuis l'enceinte contenant le matériau poreux à analyser vers les moyens utilisés pour injecter les fluides.

Ces deux risques peuvent alors conduire à fausser les résultats d'analyse sur l'échantillon de matériau poreux ou, à tout le moins, à encrasser ou perturber le fonctionnement de différents composants du dispositif d'analyse, tout particulièrement les composants utilisés pour injecter des fluides dans l'enceinte contenant l'échantillon ou pour évacuer des fluides de ladite enceinte.

Le but de l'invention est alors de proposer un dispositif d'analyse de comportement de matériau poreux amélioré, ainsi que le procédé le mettant en œuvre. Plus particulièrement, il s'agit de réduire voire supprimer tout risque de contamination biologique (micro-organisme) non voulue, aussi bien dans l'enceinte contenant l'échantillon de matériau poreux à analyser que dans les autres composants du dispositif, notamment ceux permettant l'injection et/ou l'évacuation de fluides dans/hors de l'enceinte en question.

### Résumé de l'invention

L'invention a tout d'abord pour objet un dispositif d'analyse du comportement de biofilms au sein d'un milieu poreux, ledit dispositif comprenant :
- une enceinte définissant une cavité qui est apte à recevoir le milieu poreux et qui comprend au moins une entrée d'injection de fluide et au moins une sortie d'évacuation de fluide,
   et tel que ledit dispositif d'analyse comprend aussi

- un moyen d'injection du fluide connecté fluidiquement à ladite entrée et comprenant un composant au moins pour partie logé dans l'enceinte et définissant une surface d'une première porosité P1, dite de contact, en regard du milieu poreux, ladite surface de contact étant au moins partiellement recouverte d'un filtre comportant au moins trois couches de matériau superposées, dont
- une première couche d'un matériau présentant une deuxième porosité P2 inférieure à la porosité P1 dudit composant, et qui est disposée entre
- une deuxième et une troisième couches de matériaux identiques ou différents entre eux et qui présentent des troisièmes porosités P3,P3' supérieures à la deuxième porosité P2.

Le dispositif selon l'invention peut comprendre une ou plusieurs entrées d'injection de fluide, et une ou plusieurs sorties d'évacuation de fluide

On comprend dans le présent texte par « porosité » (P1, P2 etc...) d'un matériau, la taille moyenne de ses pores. Il s'agit d'une porosité ouverte, qui permet à un fluide dont la taille des molécules qui le constituent est plus petite que la taille de la porosité du matériau en question pour pouvoir traverser ce matériau

On entend par « surface d'entrée », une surface du milieu poreux qui est en connexion fluidique avec l'entrée de l'enceinte, si bien que le fluide arrivant de l'entrée est en communication avec la surface d'entrée du milieu poreux. Avantageusement, la surface d'entrée est en regard de l'entrée de l'enceinte ou est au moins en partie logée dans l'enceinte, et fait face au milieu poreux, de façon que le fluide qui la traverse soit conduit dans ledit milieu poreux.

La solution selon l'invention consiste donc à utiliser une « tête » d'injection de fluide dont la surface en regard de l'échantillon de matériau poreux à analyser présente une porosité qui permet de « laisser » passer tout type de fluide, ou tout au moins tout type de fluide utilisé pour ce type d'analyse, et de la munir d'un filtre de porosité plus fine, qui va pouvoir filtrer/bloquer les molécules/composants dont la taille est supérieure à cette porosité plus fine.

En réglant de manière appropriée la porosité de la surface de contact et celle des couches du filtre, et notamment celle de la première couche, on peut choisir quelle molécule/micro-organisme va pouvoir passer à travers la surface de contact et quelle molécule/micro-organisme on va ensuite être filtrée par le filtre selon l'invention.

En choisissant une porosité P2 adéquate, le filtre peut ainsi bloquer les molécules/micro-organismes, biofilms, ce qui est très avantageux : on peut continuer à utiliser des têtes d'injection/des moyens d'injection en matériau conventionnel pour ce type de fonction (porosité P1), et les fonctionnaliser avec un filtre qu'on peut adapter à chaque besoin. On peut ainsi conserver le matériel d'injection connu, et lui adjoindre le filtre, ce qui est une solution simple et efficace.

Ce filtre va ainsi pouvoir garantir une meilleure étanchéité aux micro-organismes et biofilms quand cela est nécessaire, ce qui permet :
- de moins/de ne pas ou de ne plus gêner l'écoulement des fluides dans le dispositif et à travers l'échantillon de matériau poreux à analyser
- ni d'affecter les analyses faites sur l'échantillon de matériau poreux à analyser
- et de garantir une absence, ou tout au moins une réduction de « remontées » de micro-organismes, biofilms depuis l'échantillon de matériau poreux à analyser vers les autres composants du dispositif d'analyse que l'enceinte elle -même, notamment toutes les conduites, capteurs, moyens d'injection (pompe...) et d'évacuation des fluides.

Par ailleurs, il s'est avéré que le filtre est avantageusement un empilement multicouches, notamment un empilement tri-couches dont la couche centrale est celle qui assure majoritairement la fonction de filtrage. Mais du fait d'une porosité plus fine, le matériau qui la constitue tend à être moins résistant mécaniquement à des épaisseurs raisonnables, ce qui a conduit à la disposer entre deux autres couches de porosité supérieures, plus résistantes et qui vont la protéger des détériorations, notamment mécaniques.

Selon un mode de réalisation de l'invention, le composant en question peut avoir la forme d'un piston, orienté selon un axe coaxial avec un axe longitudinal de l'enceinte et qui est mobile par coulissement selon son axe ou statique, la surface de contact correspondant à la surface extérieure de la tête du piston qui est logée dans l'enceinte.

Avantageusement, ce composant fait partie de l'embout d'injection de fluide du dispositif, cet embout de prenant de préférence la forme d'un piston.

De préférence, le matériau définissant la surface de contact peut présenter une première porosité P1 d'au moins 30 micromètre, de préférence au voisinage de 35 à 45 micromètres. Cette porosité P1 est conventionnelle dans le domaine, et permet de laisser passer tous les types de fluide habituellement utilisés dans ce type d'analyse.

De préférence, le matériau de la première couche du filtre peut présenter une deuxième porosité P2 d'au plus 0,30 micromètre, notamment d'au plus 0,28 micromètre, de préférence comprise entre 0,20 et 0,25 micromètres. Avec ce type de porosité, on bloque/filtre la plupart, voire la totalité des micro-organismes les plus courants (de type bactéries).

De préférence, le matériau de la deuxième couche et/ou de la troisième couche du filtre peut présenter une troisième porosité P3, P3' d'au moins 30 micromètres, de préférence comprise entre 35 et 45 micromètre. Avantageusement les deuxième et troisième couches peuvent être constituées du même matériau et présenter la même porosité ou une porosité similaire. Ces porosités P3, P3' peuvent être proches de ou égales à la porosité P1 de la surface de contact.

On voit ainsi une très grande différence de porosité entre la première couche du filtre et les deux autres et le matériau définissant la surface de contact, avec une porosité au moins 10 fois, notamment au moins 100 fois plus faible pour cette première couche, qui est la couche qui va assumer la majorité et même la totalité de la fonction filtrante du filtre.

De préférence, les matériaux des couches du filtre sont choisis pour résister à une température d'au moins 50°C ou d'au moins 80°C, et à une pression d'au moins 10⁶ Pa (soit au moins10 bars). Ils peuvent être notamment en métal ou en alliage métallique ou en céramique ou à base de polymère(s). En effet, ils doivent pouvoir résister aux conditions de fonctionnement du dispositif d'analyse, qui reproduit au mieux les conditions réelles en milieu sous-terrain.

Selon un mode de réalisation de l'invention, le matériau de couche centrale du filtre est à base de polymère(s), par exemple de la famille des polyfluoroéthènes, notamment à base de polyfluorure de vinylidène PVDF. Ce type de matériau est en effet résistant à la chaleur et permet de filtrer efficacement les bactéries.

Selon un exemple de réalisation, le matériau de la deuxième couche du filtre et de la troisième couche du filtre est à base de polymère(s), notamment à base de polyamide(s).. Ce type de matériau est également résistant à la chaleur et présente aussi une bonne résistance mécanique à des épaisseurs raisonnables.

Les trois couches du filtre peuvent être solidarisées entre elles par des moyens de fixation mécaniques et/ou chimiques, notamment par une bague de sertissage commune venant plaquer le filtre contre la surface de contact et/ou par une colle, répartie par exemple par points de colle et/ou sous forme de joint périphérique. Elles peuvent ainsi adhérer les unes aux autres sur tout ou partie de leurs surfaces. Elles peuvent aussi être maintenues les unes contre les autres par un moyen mécanique sans adhésion entre elles.

Le filtre peut être solidarisé à la surface de contact de façon réversible ou non. Un montage réversible présente l'avantage de pouvoir le changer facilement quand il est jugé saturé, ou de pouvoir le retirer, le nettoyer puis le remettre en place si un nettoyage efficace est envisageable.

Le choix d'épaisseur des couches du filtre va dépendre des matériaux qui les constituent. De préférence, les épaisseurs sont choisies de manière à ne pas impacter trop significativement la mesure de pression, en prenant en compte leur taille de pores.

De préférence, l'épaisseur de la première couche peut être comprise entre 50 et 200 micromètres, notamment entre 80 et 170 micromètres, par exemple entre 120 et 130 micromètres.

De préférence, l'épaisseur de chacune des deuxième et troisième couches peut être comprise entre 20 et 100 micromètres, notamment entre 30 et 80 micromètres, par exemple entre 40 et 60 micromètres.

De préférence, l'épaisseur totale du filtre avec les trois couches est comprise entre 150 et 300 micromètres, notamment entre 200 et 250 micromètres, par exemple entre 215 et 235 micromètres.

Le dispositif d'analyse selon l'invention peut aussi comprendre au moins un moyen de mesure de paramètres chimiques, biologiques et/ou physiques, notamment choisi parmi au moins : un capteur de pH, un capteur de O₂, un capteur de CO₂, un capteur de H₂S, un capteur de H₂, un glucosimètre, un conductimètre, un viscosimètre, un densimètre, un capteur de température, un capteur de pression.

Ledit ou lesdits moyens de mesure peuvent notamment être disposé(s) sur une conduite en connexion fluidique avec l'entrée d'injection de fluide et/ou la ou une des sorties d'évacuation de fluide, pour faire des mesures en ligne.

Le dispositif d'analyse selon l'invention peut comprendre un dispositif de contrôle de la pression utilisant les données de capteurs de pression mesurant une différence de pression entre le fluide entrant dans l'enceinte par l'entrée d'injection de fluide et le fluide sortant de l'enceinte par la sortie d'évacuation de fluide.

Pour ce faire, le dispositif de contrôle peut comprendre notamment une ou des vannes de contre-pression.

Le dispositif d'analyse selon l'invention peut comprendre un dispositif de scanner aux rayons X apte à analyser le milieu poreux à travers les parois de l'enceinte.

Dans ce cas, le dispositif d'analyse, ou certains de ses composants au moins, ou au moins l'enceinte dans laquelle est disposé l'échantillon de matériau poreux sont en matériau transparent aux rayons X.

Le dispositif d'analyse selon l'invention peut comprendre des moyens informatiques aptes ;
- à piloter des moyens de mesure en lignes de paramètres chimiques, biologiques et/ou physiques
- et/ou à collecter et traiter les résultats de mesure desdits moyens, notamment en vue de contrôler/réguler au moins un desdits paramètres
- et/ou à collecter et traiter les résultats de mesures réalisées sur le milieu poreux, notamment par scanner aux rayons X.

Les moyens informatiques peuvent être au moins un contrôleur ou au moins un calculateur ou au moins un ordinateur.

De préférence, le dispositif d'analyse comprend une enveloppe thermostatée dans laquelle l'enceinte est positionnée. Avantageusement, ladite enceinte est cylindrique. L'enceinte peut comprendre plusieurs secteurs circonférentiels indépendants pour drainer le milieu poreux et maintenir une pression constante en périphérie.

L'invention a également pour objet un procédé d'analyse du comportement d'un biofilm au sein d'un milieu poreux mettant en œuvre le dispositif d'analyse décrit plus haut.

Ce procédé peut comprendre l'injection d'un fluide ou une succession d'injections de différents fluides à l'entrée de l'enceinte à travers le composant définissant la surface de contact de première porosité P1 et à travers le filtre qui la recouvre au moins partiellement.

Ledit ou lesdits fluides peuvent notamment être choisis parmi au moins un des fluides suivants : solution aqueuse contenant éventuellement un ou des sels minéraux, fluide contenant des nutriments pour des microorganismes, fluide comprenant des microorganismes, fluide comprenant un biocide.

Le procédé d'analyse peut prévoir de contrôler un gradient de pression entre le fluide entrant par l'entrée dans l'enceinte et le fluide sortant par une sortie de l'enceinte, de manière à imposer un écoulement du fluide à gradient de pression prédéterminé dans le milieu poreux, les détails d'un mode de réalisation pour parvenir à ce contrôle sont décrits dans le brevet FR3121452 précité.

### Liste des figures

D'autres caractéristiques et avantages du dispositif et du procédé selon l'invention, apparaîtront à la lecture de la description ci-après d'exemples non limitatifs de réalisations, en se référant aux figures annexées et décrites ci-après.
[Fig. 1]

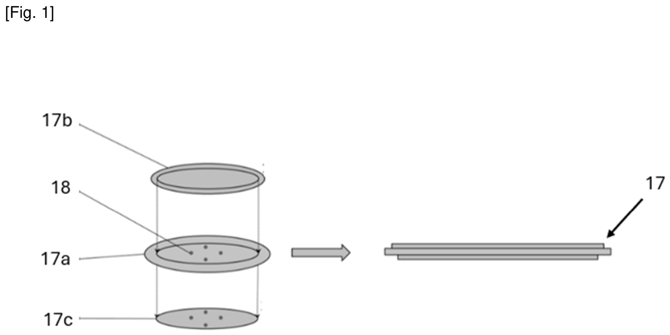

   La figure 1 un mode de réalisation d'un filtre selon l'invention.
[Fig. 2]

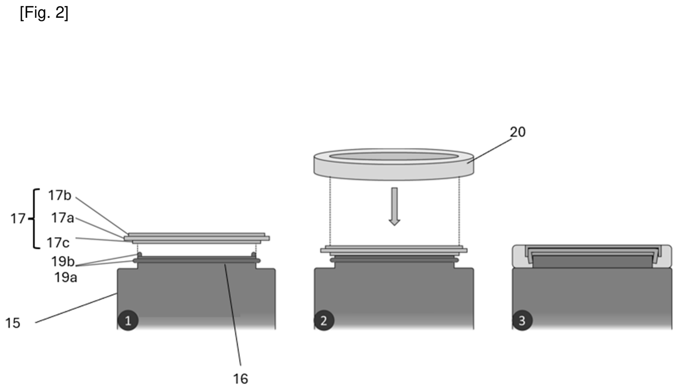

   La figure 2 représente un mode d'assemblage du filtre représenté à la figure 1 à la tête d'injection de fluide du dispositif d'analyse selon l'invention.
[Fig. 3]

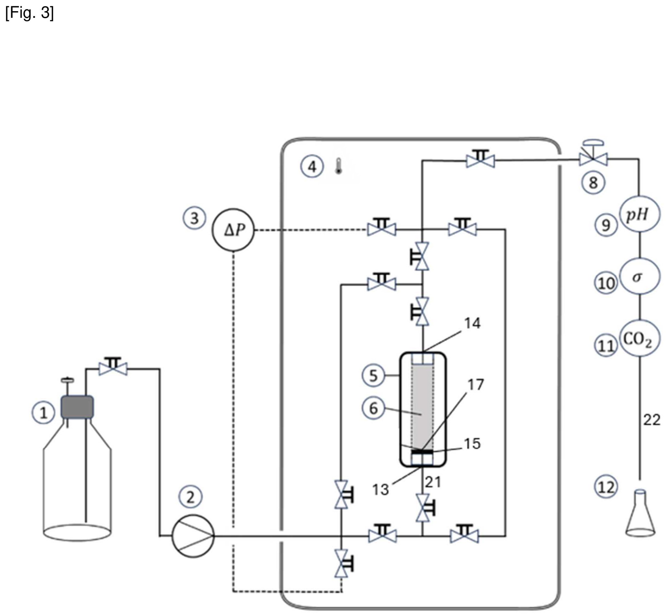

   La figure 3 représente un mode de réalisation du dispositif d'analyse de l'invention intégrant la tête d'injection munie du filtre représentée à la figure 2.

Les figures sont schématiques et les différents composants ne sont donc pas nécessairement représentés à l'échelle. Les mêmes composants gardent la même référence d'une figure à l'autre.

### Description des modes de réalisation

On entend par « biofilm » un film ou une couche de biomasse généré par des microorganismes tels que des bactéries ou des champignons. En effet, les microorganismes peuvent s'assembler et former une masse compacte formant un film. Le biofilm peut altérer la perméabilité du milieu poreux (roche réservoir, ciment du casing des puits par exemples) et limiter le débit, voire empêcher totalement l'écoulement de fluide à travers ces milieux.

On rappelle également que les bactéries sont des microorganismes unicellulaires et ne sont donc généralement pas organisés en tissus. Chaque bactérie se développe et se divise indépendamment de toute autre bactérie, bien que des agrégats de bactéries, contenant parfois des membres d'espèces différentes, soient fréquemment trouvés. Il existe plusieurs types de bactéries classées en genres et en espèces, qui varient selon leurs formes et leurs couleurs ou mêmes leurs conditions de croissance.

Dans le cadre d'exemples de mise en œuvre du dispositif selon l'invention, la bactérie «modèle » choisie peut être *Escherichia Coli* (*E. Coli*), notamment parce qu'elle survit à la fois en présence et en absence d'oxygène. Naturellement, l'invention peut s'appliquer à l'étude de toute autre bactérie ou champignon, aérobie ou anaérobie. Elle peut notamment s'appliquer à des bactéries, généralement en mélange (consortium), prélevées directement depuis les roches poreuses souterraines d'intérêt.

Les cellules d'*E*. *Coli* sont généralement en forme de bâtonnets et mesurent environ 2 micromètres de long, avec un diamètre d'environ 0,5 micromètres.

Dans des conditions optimales, les bactéries peuvent se développer et se diviser extrêmement rapidement. Le temps de division est variable selon le type de bactéries (en minutes, en heures, ou en jours).

Dans la nature, de nombreux organismes vivent dans des communautés (par exemple des biofilms) qui peuvent permettre un apport accru de nutriments et une protection contre les stress environnementaux. Les organismes contenus dans les biofilms présentent souvent des propriétés très différentes d'un même organisme dans l'état individuel ou dans l'état planctonique. Les bactéries qui se sont agrégées dans des biofilms peuvent donner des informations sur la taille de la population et l'état métabolique.

En laboratoire, les bactéries sont généralement cultivées dans des milieux solides ou liquides. Des milieux de croissances solides (gélose d'agar), préparés dans des boîtes de pétri sont utilisés pour isoler une souche bactérienne au sein de cultures en mélange. En complément, des milieux de croissance liquides sont aussi utilisés lorsque la mesure de la croissance ou de grands volumes de cellules sont nécessaires. La croissance dans les milieux liquides agités se présente sous forme de suspensions cellulaires souvent uniformes, ce qui facilite la division et le transfert de gaz (notamment d'oxygène) dans le milieu réactionnel des cultures. L'utilisation de milieux sélectifs (milieux contenant des nutriments spécifiques ajoutés ou déficients, ou contenant des antibiotiques) peut aider à identifier et/ou isoler des organismes spécifiques.

La plupart des techniques de laboratoire pour étudier la croissance des bactéries utilisent des niveaux élevés de nutriments pour produire de grandes quantités de cellules à moindre coût et rapidement. Cependant, dans les environnements naturels, les éléments nutritifs sont limités, ce qui signifie que les bactéries ne peuvent pas se reproduire indéfiniment. Cette limitation en éléments nutritifs a conduit à l'évolution de différentes stratégies de croissance.

La croissance bactérienne suit en effet quatre phases, l'entrée pour la première fois dans un environnement riche en nutriments d'une population de bactéries va permettre sa croissance :
- La première phase I est la phase de latence, une période de croissance lente pendant laquelle les cellules s'adaptent à l'environnement riche en nutriments et à une croissance rapide. La phase de latence est associée à des taux de biosynthèse élevés car les protéines nécessaires à la croissance rapide sont produites.
- La deuxième phase de croissance II est appelée phase exponentielle, marquée par une croissance exponentielle rapide. Au cours de cette phase, chaque bactérie génère deux bactéries filles, par scission binaire, à chaque génération. Un des paramètres physiques liés à cette croissance est le taux de croissance des cellules au cours de ladite phase, et le temps nécessaire pour que les cellules doublent, appelé temps de génération. Au cours de la phase exponentielle, les nutriments sont métabolisés à la vitesse maximale jusqu'à ce que l'un des nutriments soit épuisé et commence à limiter la croissance.
- La troisième phase de croissance III est la phase stationnaire qui est causée par une carence en nutriments. Les cellules réduisent leur activité métabolique (le nombre de cellules qui apparaissent est égal au nombre de cellules qui disparaissent).
- La phase finale IV est la phase de mortalité par épuisement des nutriments.

Il a été observé que la perméabilité hydraulique d'un matériau poreux peut être réduite jusqu'à trois ou quatre ordres de grandeur en raison de la formation de biofilm au sein de la porosité disponible du matériau. Ainsi, au cours de la phase I de croissance, on a observé que les microorganismes se présentent soit de façon isolée à l'interface « parois des pores - phase liquide », soit en petites colonies. Puis, dans la deuxième phase II, on observe une diminution rapide de la perméabilité, le liquide salin dans les pores est partiellement remplacé par :
- La présence des corps biologiques : exopolysaccharides (EPS), biofilm ou agrégats de bactéries.
- La formation de bulles de gaz provoquées par la saturation excessive de la solution aqueuse en produits gazeux générés par l'activité biologique des micro-organismes, tels que le CO₂, le CH₄ et l'H₂S.
- La précipitation d'insolubles sous forme de sels, tels que le fer sulfurique formé par des micro-organismes utilisant un anion sulfate comme accepteur d'électrons
- Une combinaison d'au moins deux des composés précédents.

Dans une première hypothèse, appelée «modèle à pores fermés», les pores deviennent complètement bouchés par les biofilms poreux et perméables, et la valeur minimale de la perméabilité du matériau poreux correspond à la perméabilité intrinsèque du biofilm.

Dans une deuxième hypothèse, appelée «modèle à pores ouverts», la contrainte de cisaillement exercée par le fluide sur le biofilm provoque le détachement continu des fragments de biofilm et, par conséquent, les pores ne sont jamais complètement bouchés; ici la valeur minimale de la perméabilité du matériau poreux est déterminée par l'équilibre entre croissance et détachement.

Au cours de la dernière phase IV, on observe le rétablissement partiel (à cause de la présence de biofilms qui sont constitués de polymères très visqueux souvent difficiles à balayer en totalité), ou complet de la perméabilité (si un facteur limitant vient arrêter la croissance cellulaire).

L'invention concerne donc un dispositif d'analyse du comportement d'un biofilm au sein d'un milieu poreux, avec une enceinte définissant une cavité pour recevoir le milieu poreux à analyser et qui comprend au moins une entrée d'injection de fluide et au moins une sortie d'évacuation de fluide. Il comprend aussi un moyen d'injection du fluide connecté fluidiquement à ladite entrée et comprenant un composant éventuellement au moins pour partie logé dans l'enceinte et définissant une surface d'une première porosité P1, dite de contact, en regard du milieu poreux.

La surface de contact est au moins partiellement (notamment entièrement) recouverte d'un filtre à trois couches bien particulières : une première couche d'un matériau présentant une deuxième porosité P2 inférieure à la porosité P1 dudit composant et qui est disposée entre une deuxième et une troisième couches de matériaux identiques ou différents entre eux et qui présentent des troisièmes porosités P3,P3' supérieures à la deuxième porosité P2. Naturellement, le filtre selon l'invention peut comprendre plus de trois couches.

**La** **figure 1** est une représentation d'un tel exemple de filtre selon l'invention : le filtre 17 comprend :
- une première couche 17a composée de PVDF de porosité sensiblement égale à 0,22 micromètre et d'épaisseur 125 micromètres
- une deuxième couche 17b en toile de nylon de porosité 40 micromètres et d'épaisseur 50 micromètres
- une troisième couche 17c identique à la deuxième couche 17b (même matériau, même porosité, même épaisseur).

En partie gauche de la figure, sont représentées les trois couches avant assemblage entre elles, et en partie droite les trois couches assemblées en un filtre 17.

La première couche 17a est positionnée entre les deuxième couche 17b et troisième couche 17c après assemblage, comme visible sur la partie droite de la figure.

La taille et la forme du contour des couches constituant le filtre est adaptée à la taille et à forme de la surface de contact sur laquelle le filtre est déposé. Le filtre peut ainsi être de contour circulaire, avec des couches de diamètre de l'ordre de 1 à 10 mm. Elles sont de diamètres similaires ou identiques entre elles.

Dans ce mode de réalisation, les couches sont assemblées entre elles de façon à former un empilement avec contact de préférence direct entre elles. A titre d'exemple, on vient les fixer les unes aux autres par des points de colle 18 répartis sur la surface des couches, notamment en résine de 2-cyanoacrylate. On a représenté quatre de ces points de colle. La polymérisation de la colle une fois les points de colle posés et les couches mises en contact les unes avec les autres peut prendre de quelques minutes à quelques heures suivant la colle choisie et le conditions, notamment de température.

Une fois les couches assemblées, on vient assembler le filtre ainsi constitué sur le composant d'injection, comme représenté à la **figure 2** :
Le composant 15 est en forme de tête de piston, qui définit une surface extérieure 16 dite surface de contact 16. La fixation du filtre 17 sur la surface de contact 16 se fait en trois étapes comme représenté de gauche à droite sur la figure (numéros 1,2,3 cerclés sur fond noir) :
- on vient déposer un premier cordon de résine 19a sur le bord extérieur de la surface circulaire de contact 16, par exemple en résine de 2-cyanoacrylate, puis un second 19b sur le pourtour extérieur du premier cordon
- on vient ensuite centrer le filtre 17 au-dessus des cordons de résine et on vient sertir le filtre 17 et les deux cordons de joint par une bague de sertissage 20
- la bague 20 vient ainsi plaquer le filtre 17 contre la surface de contact 16, en présentant le dimensionnement approprié pour « coiffer » le filtre et les cordons de joint.

On voit que le filtre vient recouvrir la surface extérieure « utile » 16 de la tête de piston 15, qui a un diamètre un peu supérieur à celui du filtre et qui présente un petit ressaut permettant de caler la bague de sertissage 20.

Naturellement, on peut assembler différemment le filtre au composant d'injection 15, on peut avoir une surface d'injection 16 de forme, taille, conception différentes.

On peut aussi envisager de rapporter le filtre contre ou au voisinage du composant d'injection sans le fixer directement audit moyen d'injection, tant que le fluide est contraint de le traverser en débouchant du composant d'injection.

La **figure 3** représente un exemple de dispositif d'analyse du comportement d'échantillons de matériau poreux, notamment pour l'étude de la formation de biofilms, et qui intègre la tête de piston munie du filtre comme représentée à la figure 2. La figure 3 ne représente que les composants les plus significatifs du dispositif, et est une représentation volontairement schématique, très simplifiée et pas à l''échelle, du dispositif afin d'en faciliter la compréhension.

Le dispositif comprend :
- une bouteille Schott stérile 1
- une pompe 2 hastelloy de référence QUIZIX QX6000 commercialisée par la société

### QUIZIX

- des capteurs 3 de mesure de différence de pression en ligne
- une étuve 4
- une cellule 5 (appelée aussi enceinte dans le présent texte)
- un échantillon de milieu poreux 6 disposé dans la cellule 5
- le filtre 17 de l'invention, qui est un empilement de couches à fonction anti microbienne scellé sur la surface 16 de la tête de piston 15 appartenant aux moyens d'injection de fluide (notamment liquide) du dispositif avec la pompe 2
- un moyen de régulation de contre-pression 8
- un capteur pH en ligne 9
- un conductimètre 10
- un capteur CO₂ en ligne 11
- un bécher 12 recueillant l'effluent sortant de l'enceinte
- une ligne d'injection 21 de fluide depuis la bouteille 1 dans l'enceinte 5
- une ligne d'évacuation 22 de fluide depuis l'enceinte 5 vers le bécher 12.

L'injection de fluide, ici un liquide, par les moyens d'injection se déroule de la façon suivante : la pompe 2 vient pomper une quantité donnée de liquide depuis la bouteille 1 faisant office de réservoir. Le liquide est ensuite acheminé via une conduite 21 équipée de vannes ad hoc dans la cellule 5 à travers l'échantillon poreux 6 par un embout d'injection en forme de piston 15 qui est muni d'un orifice traversant assurant la connexion fluidique avec la conduite 21 pour amener le liquide sur la surface 16 de la tête de piston. Cette surface est munie de moyens de répartition, par exemple des rainures, qui distribuent le fluide de façon qu'il sorte de la tête de piston vers l'échantillon 6 depuis la majeure partie de la tête de piston. Cette tête de piston est en outre recouverte du filtre 17 selon l'invention.

Il a été vérifié que le fonctionnement d'un tel dispositif était significativement amélioré par l'ajout de ce filtre 17, qui a effectivement empêché ou fortement retardé toute contamination via les moyens d'injection des différentes composants du dispositif dans son ensemble, aussi bien en amont qu'en aval de l'enceinte où se trouve l'échantillon de matériau poreux 6, que l'échantillon lui-même : on peut obtenir des analyses plus précises/plus fiables et éviter tout encrassement / contamination du dispositif.

Ci-dessous on rappelle certaines conditions opératoires possibles pour utiliser le dispositif, à titre d'exemples non limitatifs :
Le fluide injecté dans la cellule 5 pour traverser l'échantillon 6 peut être une solution aqueuse nutritive des microorganismes (pour favoriser la croissance des microorganismes et ainsi le biofilm dans le milieu poreux). Alternativement, le fluide injecté peut être une solution non aqueuse. Le fluide injecté peut éventuellement aussi comprendre des microorganismes pour générer le biofilm : cette configuration peut être représentative d'une situation réelle avec un fluide « contaminé » par des microorganismes qui peuvent générer involontairement un biofilm dans le milieu poreux.

Le fluide entrant peut comprendre de la saumure.

Par définition, la saumure est une solution d'eau salée faite généralement avec du chlorure de sodium (NaCl), mais on peut utiliser d'autres types de sel. La concentration en sel peut être faible ou saturée, suivant le type d'étude à réaliser. Dans le cadre de l'invention, l'exemple de saumure utilisée est constitué de NaCl dans de l'eau, à la concentration de 10 g/l afin de se rapprocher des conditions réelles.

Selon une configuration de l'invention, le fluide peut comprendre un milieu de culture (aussi appelé « solution nutritive ») composé d'un mélange de substrats nutritifs (acides aminés, peptides, bases nucléiques, sucres, etc.), d'un système tampon pour éviter les variations importantes du pH, de sels minéraux et de vitamines.

Le milieu dit « Luria Broth » est un milieu très riche en substrats (carbone, protéines, azote, extrait de levure). Sa composition après dissolution dans l'eau est la suivante : Tryptone 10 g/l, extrait de levure 5g/l, NaCl 10 g/l, le pH de la phase liquide est ajusté à environ 7.

Le milieu dit « Fouad » modifié par l'Institut Pasteur est un milieu adapté à l'enrichissement en entérobactéries comme *Escherichia Coli,* sa composition dans l'eau est indiquée dans le tableau 1 ci-dessous :

**[Table 1]**

| Composés du Milieu FOUAD modifié | Formule Chimique | Concentration |
|---|---|---|
| chlorure de sodium | NaCl | 5 g/l |
| dihydrogénophosphate de potassium | K₂HPO₄ | 5 g/l |
| Mono hydrogénophosphate de potassium | KH2PO4 | 5 g/l |
| phosphate d'ammonium | (NH₄)₃PO₄ | 2 g/l |
| phosphate de magnésium | MqSO4.7H2O | 0,2 g/l |
| sulfate de manganèse | MnSO4.4H₂O | 0,0 2g/l |
| Perchlorure de Fer | FeCl3 | 0,005 g/l |
| Extrait de levure | | 3,5 mg/l (0,0035g/l) |

Ce milieu est contraint en azote, pour mieux représenter des conditions réelles. Le milieu de culture FOUAD non contraint en azote peut contenir jusqu'à 0,5 g/l d'extrait de levure (soit environ 100 fois plus que dans le milieu préconisé ici).

Le couple K₂HPO₄/KH₂PO₄ joue un rôle de tampon afin de réguler le pH autour de la valeur du pH choisi, ici autour de 6,8.

Le fluide injecté peut également être une solution biocide prévue pour détruire les microorganismes à l'origine du biofilm. Ainsi, on peut tester l'efficacité de solutions pour détruire partiellement ou totalement le biofilm. On peut également étudier le comportement du biofilm face à ces différentes solutions. On peut utiliser des biocides suivants :
- Le THPS: Tetrakis (Hydroxymethyl) Phosphonium Sulfate est un microbicide utilisé pour le traitement de l'eau, pouvant inhiber la croissance microbienne de la plupart des micro-organismes aérobies, des microorganismes formant un biofilm dans les processus de récupération assistée du pétrole, des systèmes de production et d'injection d'eau dans les puits souterrains. On a montré que dès 60 ppm (mg/l) et en conditions planctoniques, le THPS a une action très efficace sur la croissance microbienne des bactéries sulfato-réductrices et méthanogènes. Le THPS se caractérise par son faible point de solidité et sa bonne stabilité, il peut se dissoudre facilement dans l'eau et se conserve longtemps.
- Le DBNPA : 2,2-dibromo-3-nitrilopropionamide : à 200 ppm (parties par millions), le DBNPA a un effet bactéricide sur toutes les bactéries en culture libre, le temps de réaction pour ce type de biocide est assez court (10 minutes).
- Le Glutaraldéhyde (ou Pentane-1,5-dial) est utilisé dans plusieurs domaines d'application, c'est un produit très stable (pour un pH compris entre 4 et 7). En milieu de culture libre, il inhibe de façon instantanée toutes les bactéries présentes pour une concentration à 1000 ppm. Selon une mise en œuvre de l'invention, le fluide peut contenir un ou plusieurs biocides tels que :
   Les biocides peuvent, selon le type et la concentration utilisée, avoir un effet bactériostatique (inhibition partielle ou totale de la multiplication) ou bactéricide (mort des bactéries).

Afin d'avoir une meilleure approche du choix de biocide ainsi que de la concentration et de la quantité minimale à mettre en œuvre pour prévenir et stopper les mécanismes d'encrassement lors de la croissance de biofilm dans des matériaux poreux, on peut procéder selon l'invention à une étude de la croissance bactérienne en milieu poreux puis de son évolution sous l'effet d'un biocide.

Par débit « constant », on entend un débit avec moins de 1% de variation au cours du temps. L'utilisation d'un débit constant permet de faciliter la génération du gradient de pression constant. En effet, avec un débit variable, il faudrait alors ajuster en permanence les pressions ou subir les changements de débits.

Le débit constant peut notamment être obtenu grâce à une pompe. Et, comme décrit plus en détails dans le brevet précité FR3121452 dans des variantes du dispositif d'analyse représenté à titre d'exemple à la figure 3, on peut contrôler un gradient de pression dans le milieu poreux.

Le maintien d'un gradient de pression est intéressant car il permet d'étudier les phénomènes dans des conditions représentatives des applications. On peut aussi faire varier le gradient de pression pour étudier l'effet du gradient de cisaillement dans le milieu poreux sur le biofilm.

Le dispositif de l'invention est adapté pour être mis en place dans un laboratoire et a notamment pour objectif de participer à la compréhension des phénomènes à l'origine de la création d'un biofilm dans un milieu poreux, à sa croissance dans ce milieu poreux, à sa dégénération et à sa résilience. Ces compréhensions serviront ultérieurement à contrôler la création ou la croissance de biofilms dans des milieux poreux réels naturels (par exemple des réservoirs rocheux, des sables) ou artificiels (par exemple, la cimentation de puits d'injection/production) ou à permettre plus facilement leur destruction dans ces mêmes milieux.

Les milieux poreux utilisés peuvent être consolidés ou inconsolidés.

On entend par « milieu poreux consolidés » un milieu poreux formé par un solide constitué de pores.

On entend par « milieu poreux inconsolidé » un milieu poreux formé par un assemblage de grains solides non reliés entre eux ce qui rend un écoulement des grains solides possibles. Le sable est un exemple de milieu poreux inconsolidé.

La pression en sortie de l'enceinte est de préférence inférieure ou égale à 20 bar pour faciliter les expérimentations.

De plus, la température peut être avantageusement comprise entre 13° et 100°C pour permettre des conditions de vie favorables aux microorganismes et à leur développement.

Dans une configuration avantageuse de mise en œuvre du dispositif selon l'invention, on peut réaliser des mesures en ligne en amont (avant l'entrée du fluide dans l'enceinte) et/ou en aval (après la première et/ou la deuxième sorties du fluide de l'enceinte ) de l'enceinte. Ces mesures en ligne permettent d'accéder à différentes informations du fluide avant son passage dans l'enceinte ou après son passage, notamment sa composition. On peut par exemple avantageusement mesurer la viscosité, la teneur en oxygène, en dioxyde de carbone (CO₂), en hydrogène et/ou en sulfure d'hydrogène (H₂S) du fluide, le pH, la conductimétrie, la teneur en glucose et/ou la densité. Toutes ces mesures permettent de suivre l'activité bactérienne et ses modifications. La différence de viscosité ou de densité entre l'entrée et la sortie de l'enceinte permettent d'identifier la présence de biofilm, et/ou d'EPS dans le fluide, ainsi que l'évolution de la composition des fluides injectés lors de leur passage dans le milieu poreux et ainsi d'évaluer la quantité de biofilm qui peut être présente dans le milieu poreux. La teneur en oxygène permet d'évaluer la croissance et la présence des microorganismes, notamment des microorganismes aérobies, dans le milieu poreux. En effet, si la teneur en oxygène entre l'entrée du fluide et sa sortie a diminué, on peut s'attendre à ce que les microorganismes aérobies aient consommé l'oxygène pour se reproduire. Ainsi, ce peut être le signe d'une croissance de biofilm. Il en va de même du suivi du dioxyde de carbone (CO₂), de l'hydrogène et/ou du sulfure d'hydrogène (H₂S) du fluide, du pH, de la conductimétrie, et de la teneur en glucose qui permettent de suivre le métabolisme des bactéries et du biofilm.

Selon une mise en œuvre de l'invention, on peut contrôler la température de l'enceinte, la température des milieux d'applications étant souvent constante car contrôlée par le gradient géothermique. De plus ce paramètre est susceptible d'intervenir dans la croissance ou la dégénération du biofilm. Ainsi, il est intéressant de pouvoir évaluer son effet.

De préférence, on peut analyser le milieu poreux par scanner aux rayons X. Cette mesure par scanner permet de visualiser la création, la croissance ou la dégénération du biofilm dans le milieu poreux en temps réel. Elle participe ainsi à mieux comprendre les phénomènes liés au biofilm.

Selon un premier mode de réalisation de l'invention, l'enceinte peut être cylindrique (et le milieu poreux peut être cylindrique aussi) et on peut faire traverser le fluide dans le milieu poreux de manière longitudinale depuis une entrée vers une deuxième sortie longitudinales, de préférence sur l'axe de l'enceinte. Ce premier mode de réalisation est simple et rapide à mettre en œuvre et permet d'étudier les phénomènes liés au biofilm.

Selon un deuxième mode de réalisation de l'invention, l'enceinte peut être cylindrique (et le milieu poreux peut être cylindrique ou annulaire doté d'un trou axial) et on peut faire traverser le fluide dans le milieu poreux de manière radiale, l'une de l'entrée ou de la deuxième sortie étant axiale et l'autre étant radiale. Cette configuration d'un écoulement radial est proche de l'écoulement réel dans un milieu rocheux, pour l'injection ou l'extraction de gaz tel que le CO₂ dans un réservoir pétrolier par exemple.

En outre, l'invention concerne également un dispositif d'analyse du comportement des biofilms au sein d'un milieu poreux (consolidé ou inconsolidé) conçu pour mettre en œuvre le procédé tel que décrit précédemment. Le dispositif comprend une enceinte apte à recevoir le milieu poreux, au moins une entrée pour injecter un fluide dans le dispositif d'analyse, au moins deux sorties pour faire ressortir le fluide du dispositif d'analyse. en aval du milieu poreux, dans le sens de circulation du fluide. Le premier moyen de contrôle de la pression est relié à la première sortie de l'enceinte et le deuxième moyen de contrôle de la pression est relié à la deuxième sortie de l'enceinte. De plus, le dispositif comprend également un moyen de commande pour contrôler l'un des deux moyens de contrôle de la pression en fonction de l'autre afin de maintenir un gradient de pression à une valeur prédéterminée le long du milieu poreux. Les moyens de contrôle de la pression ont pour rôle de prédéfinir une pression en entrée du milieu poreux (en amont du milieu poreux dans le sens de circulation du fluide dans l'enceinte) et en sortie du milieu poreux (en aval du milieu poreux dans le sens de circulation du fluide dans l'enceinte) et ainsi, un gradient de pression aux bornes du milieu poreux. En réglant la pression d'au moins l'un des moyens de contrôle par rapport à la pression de l'autre des moyens de contrôle de la pression, on peut définir un gradient de pression au sein du milieu poreux pour contrôler la croissance ou la dégénération du biofilm. Les moyens de contrôle de la pression sont de préférence des vannes de contre-pression.

Avantageusement, le dispositif peut comprendre une enveloppe thermostatée dans laquelle ladite enceinte est positionnée. Ainsi, on peut contrôler la température de l'enceinte et donc du milieu poreux ce qui permet de se positionner dans les conditions de température de l'application visée et/ou d'évaluer l'impact de la température sur la génération ou la dégénération de biofilm dans le milieu poreux.

A ce titre, les moyens de mesure en ligne peuvent comprendre un pH-mètre, un conductimètre, un viscosimètre, des capteurs de pression et/ou de température et des analyseurs pour connaître au moins partiellement la composition. Le conductimètre permet d'avoir accès au volume poral d'un milieu poreux par tracé de l'évolution temporelle des concentrations d'ions dans le milieu (mesure de la conductivité électrique). L'unité SI de mesure de la conductivité est le Siemens par mètre (S/m), la conductivité peut aussi s'exprimer en milliSiemens par centimètre (mS/cm).

De plus, à partir de la collecte des fluides produits, on peut déterminer les caractéristiques rhéologiques du fluide d'injection en utilisant un viscosimètre avec par exemple différents types de couples rotor-stator de géométrie «Couette». Ce viscosimètre est composé de deux cylindres concentriques : le cylindre intérieur est fixe (stator) tandis que le cylindre extérieur est mobile (rotor). L'entrefer, situé entre les deux cylindres, reçoit le fluide (porté à la température d'expérimentation). La viscosité du fluide est obtenue par mesure de la contrainte nécessaire au cylindre intérieur pour s'opposer au mouvement de rotation du fluide.

Afin de caractériser la croissance bactérienne et son activité, les moyens de mesure en ligne peuvent comprendre des appareils analytiques utilisés pour l'analyse des solutions réactionnelles:
- une sonde enzymatique de mesure de glucose GM10, pour la détermination des vitesses d'assimilation du glucose par *E. Coli* durant notre étude, cet appareil permet de contrôler la concentration en glucose (substrat bactérien) des solutions nutritives avant injection et au cours de la croissance des micro-organismes
- un spectrophotomètre « Shimadzu » permet de déterminer la concentration cellulaire des bactéries en solution, et ainsi d'accéder à la quantité de micro-organismes en solution par mesure de la densité optique à 600 nanomètres.

Avantageusement, le dispositif peut également comprendre des moyens informatiques aptes à piloter les moyens de mesure et/ou à collecter et traiter les résultats de mesure en ligne.

Selon une configuration avantageuse de l'invention, le dispositif et/ou ladite enceinte sont en matériau transparent aux rayons X, ce qui permet de pouvoir réaliser une mesure par rayons X du milieu poreux afin d'identifier les zones où le biofilm se forme et la quantité de biofilm à l'intérieur du milieu poreux. De préférence, le matériau transparent aux rayons X est en verre, un polymère ou un métal qui laisse facilement passer les rayons X. De manière encore préférée, lorsque l'enceinte comprend des secteurs circonférentiels indépendants, ces secteurs peuvent être en verre fritté. Ainsi, ils peuvent laisser passer les rayons X et permettre l'évacuation du fluide, le verre fritté étant perméable. Le verre fritté s'obtient en agglomérant des perles fines de verre de même grosseur avec un liant comme le glycérol, comprimant la pastille et détruisant le liant sous l'action de la chaleur.

## Revendications

1. Dispositif d'analyse du comportement de biofilms au sein d'un milieu poreux (6), ledit dispositif comprenant :
- une enceinte (5) définissant une cavité qui est apte à recevoir le milieu poreux (6) et qui comprend au moins une entrée (13) d'injection de fluide et au moins une sortie (14) d'évacuation de fluide,
**caractérisé en ce que** ledit dispositif d'analyse comprend aussi :
- un moyen d'injection du fluide (1) connecté fluidiquement à ladite entrée (13) et comprenant un composant (15) au moins pour partie logé dans l'enceinte et définissant une surface (16) d'une première porosité P1, dite de contact, en regard du milieu poreux (6), ladite surface de contact étant au moins partiellement recouverte d'un filtre (17) comportant au moins trois couches de matériau superposées, dont une première couche (17a) d'un matériau présentant une deuxième porosité P2 inférieure à la porosité P1 dudit composant et qui est disposée entre une deuxième et une troisième couches (17b, 17c) de matériaux identiques ou différents entre eux et qui présentent des troisièmes porosités P3,P3' supérieures à la deuxième porosité P2.

2. Dispositif d'analyse selon la revendication précédente, **caractérisé en ce que** le composant (15) a la forme d'un piston, orienté selon un axe coaxial avec un axe longitudinal de l'enceinte (5) et qui est mobile par coulissement selon son axe ou statique, la surface de contact (16) correspondant à la surface extérieure de la tête du piston qui est logée dans l'enceinte.

3. Dispositif d'analyse selon l'une des revendications précédentes, **caractérisé en ce que** le matériau définissant la surface de contact (16) présente une première porosité P1 d'au moins 30 micromètres, de préférence au voisinage de 35 à 45 micromètres.

4. Dispositif d'analyse selon l'une des revendications précédentes, **caractérisé en ce que** le matériau de la première couche (17a) du filtre (17) présente une deuxième porosité P2 d'au plus 0,30 micromètre, notamment d'au plus 0,28 micromètre, de préférence comprise entre 0, 20 et 0,25 micromètres.

5. Dispositif d'analyse selon l'une des revendications précédentes, **caractérisé en ce que** le matériau de la deuxième couche (17b) et/ou de la troisième couche (17c) du filtre (17) présente une troisième porosité P3 d'au moins 30 micromètres, de préférence comprise entre 35 et 45 micromètres.

6. Dispositif d'analyse selon l'une des revendications précédentes, **caractérisé en ce que** les matériaux des couches (17a,17b,17c) du filtre (17) résistent à une température d'au moins 50°C ou d'au moins 80°C, et à une pression d'au moins 10⁶ Pa ,en étant notamment en métal ou en alliage métallique ou en céramique ou à base de polymère(s).

7. Dispositif d'analyse selon l'une des revendications précédentes, **caractérisé en ce que** le matériau de la première couche (17a) du filtre (17) est à base de polymère(s), notamment à base de polyfluoroéthènes , de préférence à base de polyfluorure de vinylidène.

8. Dispositif d'analyse selon l'une des revendications précédentes, **caractérisé en ce que** le matériau de la deuxième couche (17b) du filtre et de la troisième couche (17c) du filtre (17) est à base de polymère(s), notamment à base de polyamide(s).

9. Dispositif d'analyse selon l'une des revendications précédentes, **caractérisé en ce que** les trois couches (17a,17b,17c) du filtre (17) sont solidarisées entre elles par des moyens de fixation mécaniques et/ou chimiques, notamment par une bague de sertissage commune venant plaquer le filtre contre la surface de contact (16) et/ou par une colle, répartie par points de colle et/ou sous forme de joint périphérique.

10. Dispositif d'analyse selon l'une des revendications précédentes, **caractérisé en ce que** l'épaisseur de la première couche (17a) est comprise entre 50 et 200 micromètres, notamment entre 80 et 170 micromètres.

11. Dispositif d'analyse selon l'une des revendications précédentes, **caractérisé en ce que** l'épaisseur de chacune des deuxième et troisième couc hes (17b, 17c) est comprise entre 20 et 100 micromètres, notamment entre 30 et 80 micromètres.

12. Dispositif d'analyse selon l'une des revendications précédentes, **caractérisé en ce que** l'épaisseur totale du filtre (17) avec les trois couches (17a, 17b, 17c) est comprise entre 150 et 300 micromètres, notamment entre 200 et 250 micromètres.

13. Dispositif d'analyse selon l'une des revendications précédentes, **caractérisé en ce qu'**il comprend au moins un moyen de mesure de paramètres chimiques, biologiques et/ou physiques, notamment choisi parmi au moins : un capteur de pH, un capteur de O₂, un capteur de CO₂, un capteur de H₂S, un capteur de H₂, un glucosimètre, un conductimètre, un viscosimètre, un densimètre, un capteur de température, un capteur de pression, ledit ou lesdits moyens de mesure étant notamment disposé(s) sur une conduite en connexion fluidique avec l'entrée (13) d'injection de fluide et/ou la sortie (14) d'évacuation de fluide pour faire des mesures en ligne.

14. Dispositif d'analyse selon l'une des revendications précédentes, **caractérisé en ce qu'**il comprend un dispositif de contrôle de la pression utilisant les données de capteurs de pression mesurant une différence de pression entre le fluide entrant dans l'enceinte (5) par l'entrée (13) d'injection de fluide et le fluide sortant de l'enceinte par la sortie (14) d'évacuation de fluide, le dispositif de contrôle comprenant notamment une ou des vannes de contre-pression.

15. Dispositif d'analyse selon l'une des revendications précédentes, **caractérisé en ce qu'**il comprend un dispositif de scanner aux rayons X apte à analyser le milieu poreux à travers les parois de l'enceinte (5).

16. Dispositif d'analyse selon l'une des revendications précédentes, **caractérisé en ce qu'**il comprend des moyens informatiques aptes
- à piloter des moyens de mesure en lignes de paramètres chimiques, biologiques et/ou physiques
- et/ou à collecter et traiter les résultats de mesure desdits moyens, notamment en vue de contrôler/réguler au moins un desdits paramètres
- et/ou à collecter et traiter les résultats de mesures réalisées sur le milieu poreux, notamment par scanner aux rayons X.

17. Procédé d'analyse du comportement d'un biofilm au sein d'un milieu poreux (6) mettant en œuvre le dispositif d'analyse selon l'une des revendications précédentes, **caractérisé en ce qu'**il comprend l'injection d'un fluide ou une succession d'injections de différents fluides à l'entrée (13) de l'enceinte (5) à travers le composant (15) définissant une surface de contact (16) de première porosité P1 et à travers le filtre (17) qui la recouvre au moins partiellement, ledit ou lesdits fluides étant choisis parmi au moins un des fluides suivants : solution aqueuse contenant éventuellement un ou des sels minéraux, fluide contenant des nutriments pour des microorganismes, fluide comprenant des microorganismes, fluide comprenant un biocide.

18. Procédé d'analyse selon la revendication précédente, **caractérisé en ce qu'**on contrôle un gradient de pression entre le fluide entrant par l'entrée (13) dans l'enceinte et le fluide sortant par une sortie (14) de l'enceinte (5), de manière à imposer un écoulement du fluide à gradient de pression prédéterminé dans le milieu poreux (6).
